# EUROPEAN PATENT APPLICATION

(11) **EP 1 918 376 A1**
(43) Date of publication of application: **07.05.2008**
(21) Application number: 06022946.5
(22) Date of filing: 03.11.2006
(51) Int. Cl.: C12N 15/11, C07K 16/28, C12Q 1/68, A61K 31/713, A61K 39/395, C07K 14/71, A61P 35/00

(54) **FGFR4 promotes cancer cell resistance in response to chemotherapeutic drugs**

(71) Applicant: Max-Planck-Gesellschaft zur Förderung der Wissenschaften e.V., 80539 München (DE)
(72) Inventor: The designation of the inventor has not yet been filed
(74) Representative: Weiss, Wolfgang

(57) **Abstract**

The present invention relates to Fibroblast-Growth Factor Receptor 4 (FGRF4) inhibitors for co-administration with a therapeutic procedure or/and agent for the prevention, alleviation or/and treatment of a hyperproliferative disorder, e.g. cancer, such as chemotherapy resistant cancer. Further, the present invention relates to a diagnostic procedure wherein expression status and/or polymorphisms of the FGFR4 gene are determined in a patient suffering from a hyperproliferative disorder, e.g. cancer. Based on the results of this determination and the status of the disorder to be treated a therapeutic protocol may be developed. Yet another subject of the present invention is a screening method.

SEQUENCE LISTING

<110>  Max-Planck-Gesellschaft zur Förderung der Wissenschaften

<120>  FGFR4 promotes cancer cell resistance in response to
       chemotherapeutic drugs

<130>  36798PEP

<140>  EP 06022946
<141>  2006-11-03

<160>  13

<170>  PatentIn version 3.3

<210>  1
<211>  21
<212>  DNA
<213>  synthetic


<220>
<221>  misc_feature
<223>  siRNA 66

<400>  1
aagagcagga gctgacagta g                                                21


<210>  2
<211>  21
<212>  DNA
<213>  synthetic


<220>
<221>  misc_feature
<223>  siRNA 74

<400>  2
cagtgctcga ccttgatagc a                                                21


<210>  3
<211>  21
<212>  DNA
<213>  synthetic


<220>
<221>  misc_feature
<223>  si RNA 70

<400>  3
aactacctgc tagatgtgct g                                                21


<210>  4
<211>  21
<212>  DNA
<213>  synthetic


<220>
<221>  misc_feature

<223>  si RNA

<400>  4
caggctcttc cggcaagtca a                                                21


<210>  5
<211>  19
<212>  DNA
<213>  synthetic


<220>
<221>  misc_feature
<223>  siRNA si 66

<400>  5
gagcaggagc tgacagagt                                                   19


<210>  6
<211>  19
<212>  DNA
<213>  synthetic


<220>
<221>  misc_feature
<223>  siRNA siCtrl

<400>  6
ctacctgcta gatgtgctg                                                   19


<210>  7
<211>  19
<212>  DNA
<213>  synthetic


<220>
<221>  misc_feature
<223>  siRNA si 74

<400>  7
gtgctcgacc ttgatagca                                                   19


<210>  8
<211>  20
<212>  DNA
<213>  synthetic


<220>
<221>  primer_bind
<222>  (1)..(20)
<223>  GAPDH forward

<400>  8
accacagtcc atgccatcac                                                  20


<210>  9
<211>  20
<212>  DNA

<213>  synthetic

<220>
<221>  primer_bind
<222>  (1)..(20)
<223>  GAPDH reverse

<400>  9
tccaccaccc tgttgctgta                                                    20

<210>  10
<211>  30
<212>  DNA
<213>  synthetic

<220>
<221>  primer_bind
<222>  (1)..(30)
<223>  Bcl-xl forward

<400>  10
agaattctgc caccatgtct cagagcaacc                                         30

<210>  11
<211>  23
<212>  DNA
<213>  synthetic

<220>
<221>  primer_bind
<222>  (1)..(23)
<223>  Bcl xl everse

<400>  11
gggtgatgtg gagctgggat gtc                                                23

<210>  12
<211>  21
<212>  DNA
<213>  synthetic

<220>
<221>  misc_feature
<223>  siRNA

<400>  12
ggcucuuccg gcaagucaat t                                                  21

<210>  13
<211>  21
<212>  DNA
<213>  synthetic

<220>
<221>  misc_feature
<223>  siRNA GL2-Luciferase

<400> 13
cguacgcgga auacuucgat t 21

## Description

The present invention relates to Fibroblast-Growth Factor Receptor 4 (FGRF4) inhibitors for co-administration with a therapeutic procedure or/and agent for the prevention, alleviation or/and treatment of a hyperproliferative disorder, e.g. cancer, such as chemotherapy resistant cancer. Further, the present invention relates to a diagnostic procedure wherein expression status or/and polymorphisms of the FGFR4 gene are determined in a patient suffering from a hyperproliferative disorder, e.g. cancer. Based on the results of this determination and the status of the disorder to be treated a therapeutic protocol may be developed. Yet another subject of the present invention is a screening method.

WO 99/37299 discloses the use of FGFR4 inhibitors for the treatment and/or prevention of disorders, particularly cancer, associated with FGFR overfunction caused by FGFR mutants. The therapeutic effect of FGFR4 inhibition is based on an inhibition of migration and invasion of tumor cells caused by an increased activity of the FGFR4-Arg388 variant. The content of this document is incorporated herein by reference.

WO 03/063893 discloses the use of FGFR agonists for the diagnosis, prevention, and/or treatment of hyperproliferative disorders. The therapeutic effect of FGFR4 activation is based on an inhibition of the migration and invasion of tumor cells caused by a decreased activity of the FGFR4-Gly388 variant. The content of this document is incorporated herein by reference.

A serious problem of current cancer therapy is the intrinsic or acquired resistance of cancer cells to one or more than one anti-cancer drugs (multi-drug resistance, MDR). For example, dividing cancer cells may acquire the ability to resist against the inhibitory effect of a particular anti-cancer treatment by selection of resistant clones. A state of the art strategy to suppress development of a drug resistance, multi-drug resistance, or/and resistance against irradiation is the initial administration of chemotherapeutic drugs or/and irradiation at high doses. However, such high dose treatment regimens are hampered by severe side effects which often limits the dose to be administered. Therefore, a complete eradication of the cancer cells, in particular of metastatic cells which are not accessible by surgery, can often not be achieved.

It was found in the context of the present invention that FGFR4 surprisingly promotes cancer cell survival under treatment with chemotherapeutic agents by increasing the resistance of cancer cells against apoptosis. This effect can be distinguished from the promotion of migration/invasion. The transduction of apoptosis signals occurs via the MAP kinase cascade whereas the transduction of migration/invasion signals occurs via different pathways.

Based upon this finding, it could be demonstrated that inhibitors of FGFR4 are capable of increasing the apoptosis sensitivity in cancer cells. Therefore, it is concluded that an FGFR4 inhibitor can enhance the therapeutic efficacy of anticancer agents or/and irradiation. Thus, doses of anticancer agents or/and irradiation can be reduced to achieve a particular therapeutic effect when administered in combination with an FGFR4 inhibitor rather than when administered alone. The co-administration of the FGFR4 inhibitor with an anticancer drug is particularly advantageous in high-dose treatment regimens (e.g. first line treatments), especially if the doses of the anticancer drugs or/and radiation has to be limited due to undesired side-effects.

Further, it is concluded that the determination of FGFR4 expression status or/and the determination of polymorphisms of the FGFR4 gene, particularly a polymorphism at position 388, may lead to improved strategies for the treatment of cancer and other hyperproliferative diseases.

A first aspect of the present invention refers to the use of an FGFR4 inhibitor for the manufacture of a medicament for the prevention, alleviation or/and treatment of a hyperproliferative disorder, in combination with a further therapeutic procedure or/and a further therapeutic agent. Preferably, the further therapeutic procedure or/and agent is an apoptosis-inducing therapeutic procedure or/and agent.

A "hyperproliferative disease" or "hyperproliferative disorder" as used herein includes any neoplasia, i.e. any abnormal new growth of tissue in animals which may depend upon a dysfunction or/and a loss of growth regulation. A hyperproliferative disease includes tumour diseases and/or cancer, e.g. solid tumors such as carcinomas, adenomas and sarcomas or non-solid tumors such as leukemias. The hyperproliferative disorder is preferably characterized by FGFR4 overexpression, i.e. an increased FGFR4 expression compared to normal tissue.

In the present invention, the hyperproliferative disorder is preferably cancer, more preferably the hyperproliferative disorder is selected from kidney, bladder, brain, esophagus, gastric, pancreas, small intestine, colon, breast, lung, liver, spleen, thyroid, pituitary, adrenal, ovarian, cervix, testis, prostate cancer, glioma, melanoma, or/and leukemia.

In a preferred embodiment, the cancer is a non-metastatic cancer, particularly a non-metastatic cancer in patients having the FGFR4-388Gly allele or/and the FGFR4-388Arg allele. In a further embodiment, the cancer is a metastatic cancer, particularly a metastatic cancer in patients having the FGFR4-388 Arg allele.

In case of a non-metastatic cancer the invention is suitable for increasing the apoptosis sensitivity of a solid or non-solid cancer and thus increasing the efficacy of administered anticancer drugs and/or irradiation.

In case of a metastatic cancer, the invention is suitable for the treatment of metastases, metastases formation or/and metastases progression, in particular in micrometastases, preferably in the hyperproliferative disorders as defined above. Metastases, in particular micrometastases, are often not accessible by surgery and need pharmacological or/and irradiation treatment. The fact that the co-administration with an FGFR4 inhibitor increases the efficacy of a dosage of anti-cancer drugs or/and irradiation increases the chances of successful eradication or metastases.

In a preferred embodiment of the present invention, the hyperproliferative disorder is an at least partially therapy-resistant hyperproliferative disorder. More preferably the hyperproliferative disorder is at least partially resistant against an apoptosis-inducing therapeutic procedure or/and agent.

"Therapy resistant" in the context of the present invention includes the inability of hyperproliferative cells/cancer cells to respond to therapy, i.e. the inability of a therapeutic treatment to reduce or suppress cell proliferation or/and to induce cell death in hyperproliferative cells/cancer cells.

A serious form of therapy resistance also included herein in multi-drug resistance, as discussed above. "Therapy resistant" also includes resistance of a hyperproliferative disease against a monotherapy, in particular with a cytostatic, cytotoxic or/and chemotherapeutic agent such as an apoptosis-inducing agent or by irradiation therapy. "Therapy resistant" also includes resistance to a combination treatment of at least two selected from cytostatic, cytotoxic and chemotherapeutic agents and irradiation.

"At least partially therapy resistant" as used herein includes a reduced or suppressed responsiveness of hyperproliferative cells/cancer cells to particular treatment regimens, whereas the cells may retain their full responsiveness to other treatments. Such a particular treatment regimen may be a treatment with a first anti-cancer drug, and the hyperproliferative cells/cancer cells regarded to be "at least partially therapy resistant" have a reduced or suppressed responsiveness to this particular drug treatment, whereas the hyperproliferative cells/cancer cells still responds to other treatment regimens, such as the treatment with a second anti-cancer drug. Therapy resistance may increase with time. Therefore, "at least partially therapy resistant" as used herein also includes temporal development of therapy resistance from full responsiveness to an anti-cancer treatment regimen to complete resistance against this treatment.

The therapeutic procedure of the present invention to be combined with the FGFR4 inhibitor includes any suitable therapeutic procedure suitable for the prevention, alleviation or/and treatment of a hyperproliferative disorder, in particular suitable for the induction of apoptosis in hyperproliferative cells/cancer cells. Thus the therapeutic procedure includes a treatment with an agent for the prevention, alleviation or/and treatment of a hyperproliferative disorder (with an anti-cancer drug, in particular with an apoptosis inducing agent) or/and irradiation. In a preferred embodiment, the therapeutic procedure for the prevention, alleviation or/and treatment of a hyperproliferative disorder is irradiation therapy, more preferably a gamma irradiation therapy. Radiation treatment regimens are known by a person skilled in the art. The dosage of state of the art cancer treatments may be reduced when combined with administration of an FGFR4 inhibitor.

The agent for the prevention, alleviation or/and treatment of a hyperproliferative disorder to be combined with the FGFR4 inhibitor of the present invention is selected from cytostatic, cytotoxic or/and chemotherapeutic agents, pharmaceutically acceptable salts and derivatives thereof, and combinations thereof. Preferably, the agent is selected from doxorubicin, taxanes, such as paclitaxel or docelaxel, platinum compounds, such as cis-platin, trans-platin or oxaliplatin, nucleoside analogues such as 5-fluorouracil, or fludara, mitomycin D, etoposide, cyclophosphamide, topoisomerase inhibitors such as camptothecin, topotecan or irinotecan, proteins such as anti-tumor antibodies, pharmaceutically acceptable salts and derivatives thereof, and combinations thereof. The combinations comprise two or more active ingredients selected from the above-defined agents for the prevention, alleviation or/and treatment of a hyperproliferative disorder.

Co-administration (also termed combination treatment or combination therapy) as used herein includes the administration of an FGFR4 inhibitor of the present invention and a therapeutic procedure or/and an agent for the prevention, alleviation or/and treatment of a hyperproliferative disorder, preferably an apoptosis-inducing therapeutic procedure or/and an apoptosis-inducing agent, so that they are active at the same time.

Co-administration includes the administration of the FGFR4 inhibitor and the agent for the prevention, alleviation or/and treatment of a hyperproliferative disorder in the form of a single composition or in the form of distinct compositions, preferably at least two distinct compositions, more preferable two distinct compositions. Co-administration includes the administration of the therapeutic procedure or/and the agent for the prevention, alleviation or/and treatment of a hyperproliferative disorder and the FGFR4 inhibitor simultaneously (i.e. at the same time) or sequentially, i.e. at intervals.

Co-administration "at intervals" includes the administration of the therapeutic procedure or/and the agent for the prevention, alleviation or/and treatment of a hyperproliferative disorder and the FGFR4 inhibitor within an interval of 1 h at the maximum, 6 h at the maximum, 12 h at the maximum, 1 day at the maximum or 1 month at the maximum. Co-administration at intervals also includes the administration of the therapeutic procedure or/and the agent and the FGFR4 inhibitor with the same or with different schedules on a daily, weekly or monthly basis, which may also depend on the dosage forms of the agent and the FGFR4 inhibitor, which may be identical or different, e.g. fast release dosage forms, controlled release dosage forms or/and depot forms. For example, co-administration includes different schedules for administration of an apoptosis-inducing agent as defined above or/and irradiation treatment and the FGFR4 inhibitor.

The present invention demonstrates that FGFR4 overexpression in cancer cells can mediate an increased resistance against apoptosis induction, e.g. against chemotherapeutic agents such as doxorubicin or by irradiation, compared with cell exhibiting normal FGFR4 expression. Based on this finding, it is demonstrated that inhibition of FGFR4 increases sensitivity to apoptosis induction of cancer cells. A further finding is that FGFR4 positively regulates expression and/or activity of BclX_{L} and expression of MAP kinases, particularly Erk1 and Erk2.

Without wishing to be bound by theory, the data of the present invention indicate that one possible mechanism of drug/multidrug resistance in cancer cells is FGFR4 overexpression by which a signalling cascade is generated leading to overexpression and/or increased activity of BclX_{L} and MAP kinases, such as Erk1 and Erk2. Therefore in another embodiment of the present invention, the hyperproliferative disorder of the present invention is associated with FGFR4 overexpression and the signal cascade generated thereby.

A further aspect of the present invention relates to the use of an FGFR4 inhibitor for the manufacture of a medicament for increasing the efficacy of a therapy or/and agent against a hyperproliferative disorder, in particular of an apoptosis-inducing therapy or/and agent.

Yet another aspect of the present invention relates to the use of an FGFR4 inhibitor for the manufacture of a medicament for increasing the sensitivity of a hyperproliferative disorder against irradiation and/or medicament treatment.

In yet another embodiment of the present invention, the FGFR4 inhibition is a specific inhibition. "Specific inhibition" is a selective inhibition of the FGFR4 activity. In a specific inhibition of FGFR4, the activity of other cellular components, in particular receptors (such as FGFR1, FGFR2 or/and FGFR3) is not significantly inhibited, i.e. the IC50 values of the specific FGFR4 inhibitor to other cellular components are at least a factor of 10, preferably a factor of 100, more preferably a factor of 1000 larger than the IC50 values to FGFR4. The present invention also refers to non-specific inhibition of FGFR4.

The FGFR4 inhibitor of the present invention is preferably a molecule binding to the extracellular domain of FGFR4.

The FGFR4 inhibitor of the present invention may be a direct inhibitor or an indirect inhibitor. A direct inhibitor of FGFR4 directly inhibits FGFR4, an FGFR4 transcript or/and the FGFR4 gene, thereby reducing the FGFR4 activity.

An indirect FGFR4 inhibitor does not directly inhibit FGFR4 as described above, but on a target which interacts with FGFR4, e.g. a FGFR4 ligand, such as FGF1, -2, -4, -6, -8, -16, -17, -18 or -19, a downstream target, such as BclX_{L} or a protein in the MAP kinase cascade, such as Erk1 or/and Erk2. Furthermore, an indirect inhibition of FGFR4 may be effected by transcriptional or translational inhibitors or inhibitors of transport ways or lipid rafts.

"FGFR4 activity" as used herein includes the capability of an FGFR4 polypeptide to generate a physiological or pathophysiological effect, particularly a resistance of a cell against apoptosis.

In the present invention, the activity of FGFR4 may be inhibited on the nucleic acid level, e.g. on the gene level or on the transcription level. Inhibition on the gene level may comprise a partial or complete gene inactivation, e.g. by gene disruption. Inhibition may also occur on the transcript level, e.g. by administration of anti-sense molecules, ribozymes, siRNA molecules, which may be directed against FGFR4 mRNA, FGFR4 ligand mRNA or/and against mRNA of a downstream target. Suitable anti-sense molecules may be selected from DNA molecules, RNA molecules and nucleic acid analogues. Ribozymes may be selected from RNA molecules and nucleic acid analogues. Small double-stranded RNA molecules capable of RNA interference (siRNA molecules) may be selected from RNA molecules and nucleic acid analogues. Preferred double-stranded siRNA molecules have a strand length of 19-25 nucleotides and optionally at least one 3'-overhang. Suitable siRNA molecules may e.g. be obtained according to Elbashir et al. (Nature 411 (2001), 494-498), Elbashir et al. (Genes & Dev. 15 (2001), 188-200) or Elbashir et al. (EMBO J.20 (2001), 6877-6898). The content of these documents is herein incorporated by reference.

Further, the FGFR4 activity may be inhibited on the protein level, e.g. by administration of compounds which result in a specific FGFR4 inhibition. The inhibition on the protein level may comprise, for example, the application of antibodies or antibody fragments. In particular, these antibodies or antibody fragments are directed against FGFR4, preferably against the extracellular domain of FGFR4, or against FGFR4 ligands or/and against downstream targets. The antibodies may be polyclonal antibodies or monoclonal antibodies, recombinant antibodies, e.g. single chain antibodies or fragments of such antibodies which contain at least one antigen-binding site, e.g. proteolytic antibody fragments such as Fab, Fab' or F(ab')₂ fragments or recombinant antibody fragments such as scFv fragments. For therapeutic purposes, particularly for the treatment of humans, the administration of chimeric antibodies, humanized antibodies or human antibodies is especially preferred.

Furthermore, soluble FGFR4 receptors, e.g. receptor fragments without the membrane anchor domain, antagonistic FGFR4 ligand muteins, such as muteins of FGF1, FGF2, FGF4, FGF6, FGF8, FGF9, FGF16, FGF17, FGF18 or FGF19, peptides or low-molecular weight FGFR4 inhibitors may be used in the present invention. Examples of low-molecular weight inhibitors of FGFR4 are indolinones (Mohammadi et al. (1997), Science 276:955-960), SU5402, SU4984, and PD17304 (Koziczak et al. (2004), Biol. Chem.279, 50004-50011).

Examples of suitable siRNA molecules capable of interfering with FGFR4 mRNA and thereby knocking down FGFR4 activity are shown in Table 1. It should be noted that in the Table the sense-strands of the siRNA molecules and the respective positions of the starting nucleotides on the FGFR4 mRNA (NCBI L03840) are indicated.

**Table 1:**

| Position | | |
|---|---|---|
| aagagcaggagctgacagtag | 292 | (siRNA 66) |
| cagtgctcgaccttgatagca | 2650 | (siRNA 74) |
| aactacctgctagatgtgctg | 876 | (siRNA 70) |
| | | |
| caggctcttccggcaagtcaa | 1435 | |

The invention also encompasses the administration of at least two FGFR4 inhibitors as defined above, in particular two, three, four, or five FGFR4 inhibitors.

Further FGFR4 inhibitors may be identified by screening procedures as outlined in detail below.

Yet another aspect of the present invention is a pharmaceutical composition or kit comprising
(a) an FGFR4 inhibitor as defined above, and
(b) an agent for the prevention, alleviation or/and treatment of a hyperproliferative disorder, which is different from (a)
optionally together with a pharmaceutically acceptable carrier, diluent or/and adjuvant.

Preferably, in the pharmaceutical composition of kit of the present invention, the agent for the prevention, alleviation or/and treatment of a hyperproliferative disorder is an apoptosis-inducing agent, e.g. a chemotherapeutic agent as described above.

Pharmaceutical compositions or kits suitable for use in the present invention include compositions or kits wherein the active ingredients are contained in an effective amount to achieve its intended purpose. A therapeutically effective dose refers to that amount of the compounds that results in amelioration of symptoms or a prolongation of survival in a patient suffering from a hyperproliferative disease as defined above, in particular that results in a synergistic effect of the FGFR4 inhibitor and the agent or/and therapeutic procedure for the prevention, alleviation or/and treatment of a hyperproliferative disorder as defined above. In particular, the synergistic effect is the dosage reduction or/and the increased efficacy of the agent or/and therapeutic procedure for the prevention, alleviation or/and treatment of a hyperproliferative disorder in the co-administration as described above.

Toxicity and therapeutic efficacy of the FGFR4 inhibitor and the agent for the prevention, alleviation or/and treatment of a hyperproliferative disorder can be determined by standard pharmaceutical procedures in cell cultures or experimental animals, e.g. for determining the LD50 (the dose lethal to 50% of the population) and the ED50 (the dose therapeutically effective in 50% of the population). For any compound used in the present invention, the therapeutically effective dose can be estimated initially from cell culture assays. For example, a dose can be formulated in animal models to achieve a circulating concentration range that includes the IC50 as determined in cell culture (i.e. the concentration of the test compound which achieves a half-maximal inhibition of the growth-factor receptor activity). Such information can be used to more accurately determine useful doses in humans. The dose ratio between toxic and therapeutic effects is the therapeutic index and it can be expressed as the ratio between LD50 and ED50. Compounds which exhibit high therapeutic indices are preferred. The exact formulation, route of administration and dosage can be chosen by the individual physician in view of the patient's condition (see e.g. Fingl et al., 1975, in "The Pharmacological Basis of Therapeutics", Ch. 1, p. 1). Dosage amount and interval may be adjusted individually to provide plasma levels of the active moiety which are sufficient to maintain the receptor modulating effects, or minimal effective concentration (MEC). The MEC will vary for each compound but can be estimated from in vitro data, e.g. the concentration necessary to achieve a 50-90% inhibition of the receptor using the assays described herein. Compounds should be administered using a regimen which maintains plasma levels above the MEC for 10-90% of the time, preferably between 30-90% and most preferably between 50-90%. Dosages necessary to achieve the MEC will depend on individual characteristics and route administration. In cases of local administration or selective uptake, the effective local concentration of the drug may not be related to plasma concentration.

The actual amount of the pharmaceutical composition or kit administered is dependent on the subject being treated, on the subject's weight, the severity of the affliction, the administration route and the judgement of the prescribing physician. For antibodies or therapeutical active nucleic acid molecules, and other compounds e.g. a daily dosage of 0,001 to 100 mg/kg, particularly 0.01 to 10 mg/kg per day is suitable.

Suitable routes of administration include, for example, oral, rectal, transmucosal or intestinal administration; parenteral delivery, including intramuscular, subcutaneous, intramedullary injections, as well as intrathecal, direct intraventricular, intravenous, intraperitoneal, intranasal or intraocular injections.

Alternatively, one may administer the pharmaceutical composition or kit of the present invention in a local rather than a systematic manner, for example, via injection of the compound directly into a solid tumour, often in a depot or sustained release formulation.

Furthermore, one may administer the pharmaceutical composition or kit of the present invention in a targeted drug delivery system, for example in a liposome coated with a tumour-specific antibody. The liposomes will be targeted to and taken up selectively by the tumour.

In the pharmaceutical composition or kit of the present invention, the FGFR4 inhibitor of (a) and the agent (b) for the prevention, alleviation or/and treatment of a hyperproliferative disorder may be provided in one pharmaceutical composition (single dose form) or may be provided in different compositions (separate dose form), preferably at least two distinct compositions, more preferably two distinct compositions. In particular, the distinct compositions of the pharmaceutical composition or kit of the present invention may be administered by the same route or by different routes. Co-administration of the pharmaceutical composition or kit with a therapeutic procedure, e.g. with an apoptosis-inducing therapeutic procedure such as irradiation therapy, is as defined above.

Yet another aspect of the present invention is a method for preventing, alleviating or/and treating of a hyperproliferative disorder comprising administrating an effective amount of an FGFR4 inhibitor in co-administration with a therapeutic procedure or/and agent for preventing, alleviating or/and treating of a hyperproliferative disorder to a subject in need thereof. Preferably, in the method, the therapeutic procedure or/and agent for preventing, alleviating or/and treating of a hyperproliferative disorder is an apoptosis-inducing therapeutic procedure or/and agent. Particular embodiments of the method concern a hyperproliferative disorder, an FGFR4 inhibitor, an apoptosis-inducing therapeutic procedure, an apoptosis-inducing agent or/and modes of co-administration of the therapeutic procedure or/and the agent together with the FGFR4 inhibitor as described above. In a preferred embodiment of the method of prevention, alleviation or/and treatment, the hyperproliferative disorder is an at least partially therapy resistant hyperproliferative disorder.

The subject in need of prevention, alleviation or/and treatment may be any animal which may suffer from a hyperproliferative disorder, in particular a mammal, more particularly a human being.

A further aspect of the invention refers to the diagnosis of a hyperproliferative disorder, wherein a sample from a subject, which may suffer from a hyperproliferative disorder, is analysed for the expression of the FGFR4 gene. The sample may e.g. be a body fluid sample or a tissue sample, e.g. a biopsy. The FGFR4 expression may be determined on the nucleic acid level or on the protein level according to standard methods, e.g. using a gene array or immunochemical, i.e. immunohistochemical methods. An overexpression of FGFR4 is determined by comparing the FGFR4 expression in the sample to be analysed with the FGFR4 expression in control samples, e.g. samples from healthy subjects or with standard values.

Still a further aspect of the invention refers to the diagnosis of a hyperproliferative disorder, comprising a determination of FGFR4 polymorphisms in a sample from a subject. This determination may be performed, e.g. on the nucleic acid level according to standard methods, such as restriction fragment length polymorphism (RFLP) determination. In an especially preferred embodiment a polymorphism analysis of FGFR4-388 Gly and FGFR4-388 Arg alleles is carried out.

Still a further aspect of the invention refers to the diagnosis of a hyperproliferative disorder comprising an analysis of FGFR4 expression and a determination of FGFR4 polymorphisms.

Based on the results of the analysis of the FGFR4 expression or/and the determination of FGFR4 polymorphisms a therapeutic regimen for the treatment of the hyperproliferative disorder may designed. For the treatment of non-metastatic tumors characterized by FGFR4 overexpression, the therapeutic regimen may comprise co-administration of an FGFR4 inhibitor in combination with irradiation and/or chemotherapy as described above. Also, metastatic tumors characterized by overexpression of the FGFR4-388 Arg allele may be treated by administration of a FGFR4 inhibitor in combination with e.g. irradiation or/and chemotherapy. On the other hand, tumor metastases in a subject having the FGFR4-388 Gly allele may be treated by activation of FGFR4 as disclosed in WO 03/063893.

Yet another aspect of the present invention is a screening method for identifying or/and characterizing a compound suitable for reducing the apoptosis resistance of hyperproliferative cells, which screening method comprises determining, if the compound is a FGFR4 inhibitor.

In a particular embodiment, in the screening method of the present invention identifying the FGFR4 inhibitor comprises the steps
(a) contacting at least one compounds with FGFR4, and
(b) determining FGRF4 activity in the presence of the compound.

The screening method of the present invention may comprise the use of isolated proteins, cell extracts, recombinant cells or/and transgenic non-human animals. In particular, the FGFR4 inhibitor may be identified in a molecular or/and cellular assay.

The recombinant cells or/and transgenic non-human animals preferably exhibit an altered FGFR4 expression, in particular an FGFR4 overexpression compared to a corresponding wild-type cell or animal.

In the screening method of the present invention the FGFR4 inhibitor is preferably an inhibitor of positive BclX_{L} and/or MAP kinase regulation by FGFR4, or/and an inhibitor of FGFR4 induced resistance to apoptosis. Thus, as indicated above, step (b) may comprise determination of the amount of FGFR4, determination of BclX_{L} or/and MAP kinase upregulation by FGFR4 or/and FGFR4 induced resistance to apoptosis induction.

The screening method of the present invention can be carried out in a high-throughput format for identifying novel compounds or classes of compounds. Further, the screening method of the present invention is suitable as a validation procedure for characterizing the pharmaceutical efficacy and/or the side effects of known compounds.

Furthermore, the invention shall be explained by the following figures and example.

### Figure Legends

- Fig. 1:: FGFR4 is upregulated in doxorubicin (DXR) treated MDA-MB-453 clones.

A, The expression of FGFR4 by array analysis. Mean gene expression of FGFR4 in 10 independent experiments with cell line MDA-MB-453 and resistant clones thereof (453R). B, Expression of each resistant clone (453R1-R29) was assembled by three independent experiments.
- Fig. 2:: FGFR4 expression affects chemoresistance of breast cancer cells.

A, Immunoprecipitation of FGFR4 from MCF7-FGFR4- and MCF7-Mock cell lysates using monoclonal FGFR4 antibody and probing the blot with polyclonal FGFR4 antibody. MCF7-FGFR4 and MCF7-Mock cells were treated with 2 µM DXR for 48 hours to induce apoptosis. Propidium lodide stained cells were quantified by FACS. B, lmmunoprecipitation of FGFR4 in 453R1 knockdown cells. Knockdown was effected with 453R1. C, Dose/Response assay with different concentrations of DXR for 24 hours and measurement of apoptotic cells with Propidium lodide staining followed by FACS. D, Induction of apoptosis with the chemotherapeutic drugs Cyclophosphamide (CPA), Taxotere (TXT) and Cis-platin (CP) for 48 hours. Rate of apoptosis was measured by propidium iodide staining followed by FACS.

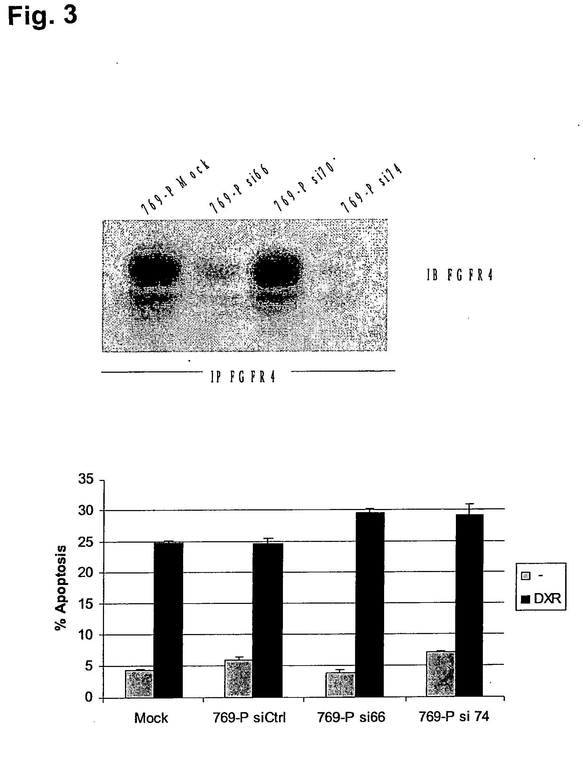

- Fig. 3:: Knockdown of FGFR4 in the colon cancer cell line 769-P increases apoptosis sensitivity.

Immunoprecipitation of FGFR4 in 769-P knockdown cells. Knockdown was effected with siRNA molecules 66, 74 and 70, respectively (cf. Table 1). Propidium iodide staining of DXR treated (2µM for 48h) 769-P cells and flow cytometry analysis were carried out for apoptosis quantification.

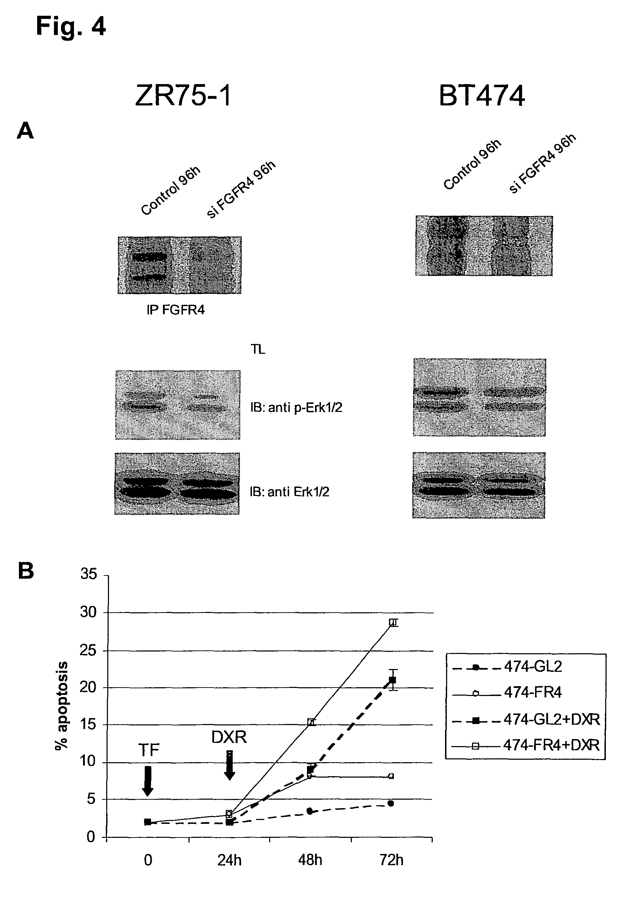

- Fig. 4:: Analysis of downstream signalling pathways in transient FGFR4 knockdown cells.

A, lmmunoprecipitation of FGFR4 in BT474 and ZR75-1 cells transiently transfected with Luciferase (control) or FGFR4 siRNA (siFGFR4). TL Immunoblots were done to monitor Erk phosphorylation. B, Apoptosis assays of transient FGFR4 knock down treated with DXR for indicated timepoints.

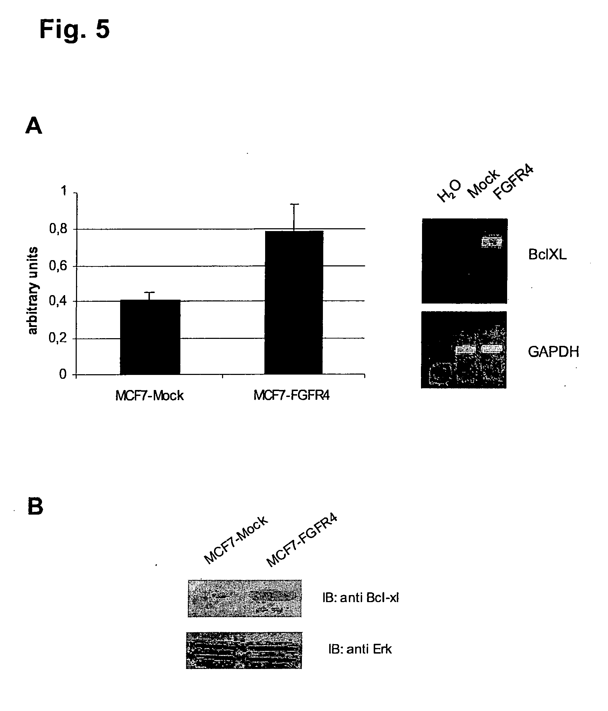

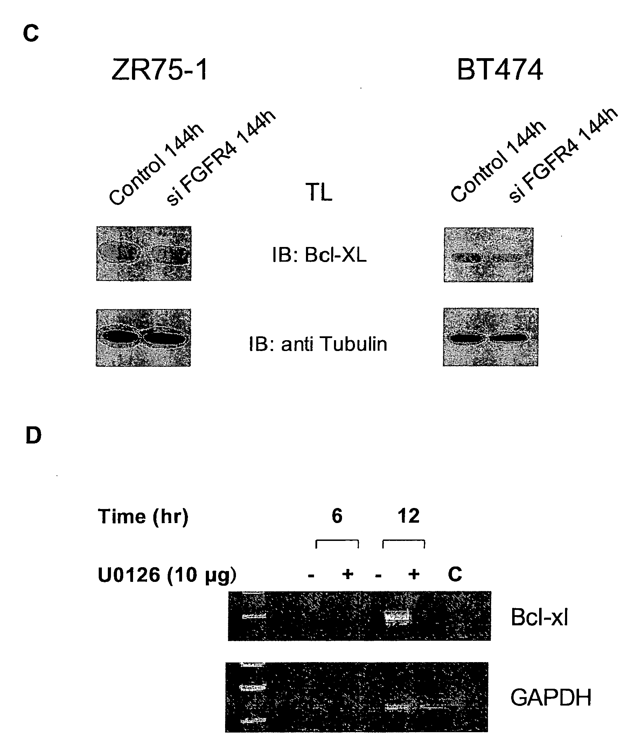

- Fig. 5:: FGFR4 affects Bcl-xl expression in different cell lines.

A, Array analysis of Bcl-xl expression in MCF7 Mock and ectopically expressing FGFR4 cells. B, TL Immunoblot of Bcl-xl expression in MCF7 cells. C, FGFR4 knockdown in BT474 and ZR75-1 cells and TL Immunoblot of Bcl-xl. D, MCF7-FGFR4 cells were treated with 10 µM U0126 for 6 hours and 12 hours. The cDNA of U0126 treated cells was utilized to perform RT-PCR with Bcl-xl primers. The integrity and amount of cDNA utilised in each RT-PCR was measured by GAPDH amplification.

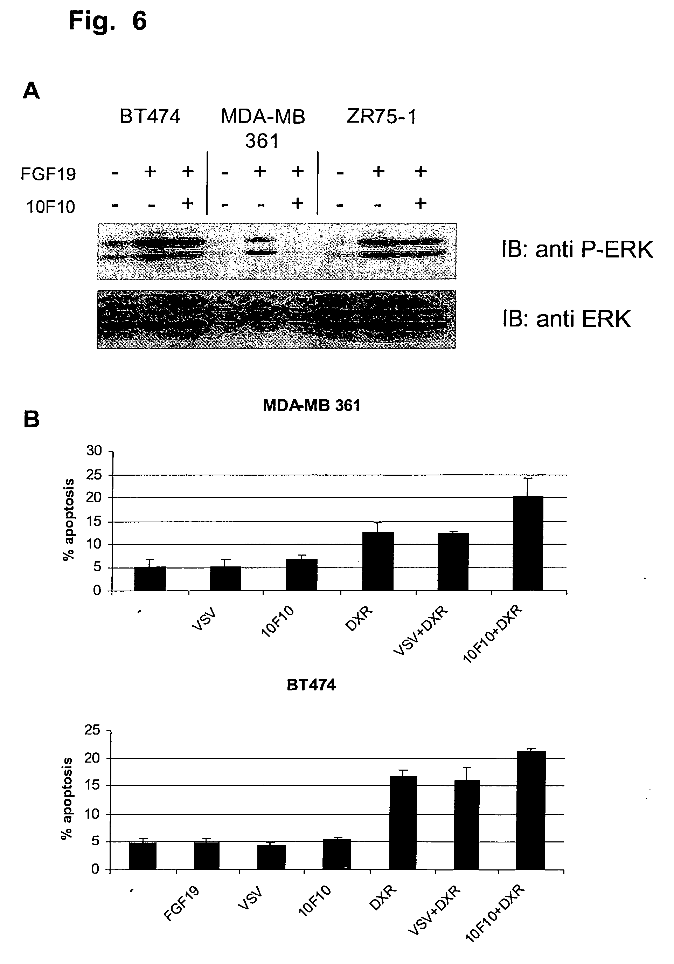

- Fig. 6:: Usage of a FGFR4 blocking antibody mediates inhibition of FGF19 induced MAPK activation.

A, Different breast cancer cell lines (BT-474, MDA-MB-361 and ZR75-1) were starved 48 h with 0% FCS/Medium. Cells were treated either with FGF19 alone or 10F10 plus FGF19 and compared with the untreated control cells. Stimulation and again inhibition of the cells is shown by a TL Immunoblot with P-ERK-AB. B, Apoptosis rate of MDA-MB361 and BT474 breast cancer cell line treated with DXR and different stimuli.

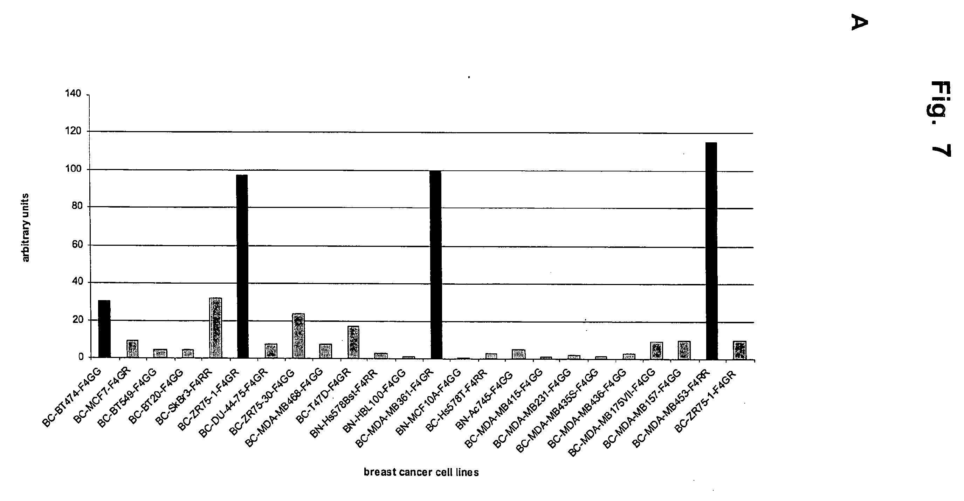

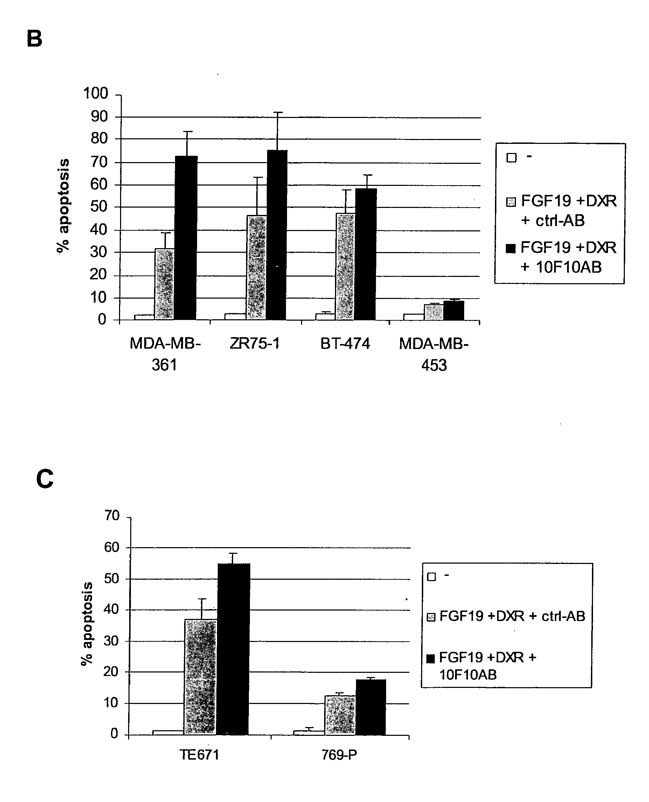

- Fig. 7:: A FGFR4 blocking antibody (10F10) decreases the chemoresistance of DXR-treated cancer cells.

A, cDNA-microarray analysis shows the relative FGFR4 expression in various breast cancer cell lines. B, Induction of apoptosis with DXR in FGF19 or FGF19/10F10 treated breast cancer cell lines showing highest FGFR4 expression. Quantification of the apoptosis rates by PJ-staining and subsequent FACS analysis. C, Apoptosis rate of two cancer cell lines with other origin than mammary gland assessed as described above.

### Example

### Materials and methods

### Reagents and antibodies

FGF-Ligands were purchased from TEBU (Offenbach, Germany). The antibodies used were polyclonal anti-FGFR4, anti-Erk2 and anti-Akt1/2 (Santa Cruz Biotechnology, Santa Cruz, CA), rabbit polyclonal anti-phospho-Akt (Ser473) (New England BioLabs, Beverly, MA), mouse monoclonal anti-phosphotyrosine antibody 4G10 (Upstate Biotechnology, Lake Placid, NY), mouse monoclonal anti-tubulin (Sigma, St Louis, MO), rabbit polyclonal anti-phospho-Erk (Cell signalling, USA) and mouse monoclonal anti-Bcl-xl (BD Biosciences, Heidelberg, Germany), mouse monoclonal anti-VSV antibody, mouse monoclonal anti-FGFR4 antibody 10F10 (U3pharma, Martinsried).

Secondary HRP-conjugated antibodies were goat anti-mouse (Sigma, Taufkirchen, Germany) and goat anti-rabbit (Biorad, Munich, Germany). If required, cells were starved by total serum withdrawal for 48 h and treated with growth factors as indicated in the figure legends.

### Cell culture, plasmids and retroviral infections

Cell lines 769-P, TE671, MDA-MB-453, BT474, MDA-MB361, MDA-MB231,ZR75-1 and MCF7 were obtained from ATCC (American type culture collection, Manassas, VA) and cultivated following the supplier's instruction except MDA-MB-453 cells, which were maintained in RPMI medium, supplemented with 10% fetal calf serum (FCS) and glutamine (Gibco/Invitrogen, Karlsruhe, Germany).

pLXSN vectors containing human fgfr4 -encoding cDNA have been described before (Bange 2002). The polyclonal MCF7 cell lines expressing FGFR4 were generated by retroviral gene transfer. Amphotropic retroviral supernatants were produced by transfection of phoenix packaging cells using the appropriate vector constructs by the calcium phosphate/chloroquine method, as described previously by Pear et al. 1993, Kinsella and Nolan 1996. At 48h post transfection, the tissue culture medium was filtered through a 0.45 µm filter, mixed with polybrene (4 µg/ml final) and used for infection of the cells. Cells were infected three times for at least 4 h and allowed to recover for 24 h with fresh medium. Polyclonal cells stably expressing FGFR4 were selected with G418 (2mg/ml) for 2 weeks.

To target FGFR4 protein for downregulation by siRNA, we generated the pRetroSuper-FGFR4 constructs according to supplier's instructions (OligoEngine). The target sequences of siRNAs used in this paper were: GAGCAGGAGCTGACAGAGT (si 66), CTACCTGCTAGATGTGCTG (siCtrl) and GTGCTCGACCTTGATAGCA (si 74). Polyclonal cells carrying pRetroSuper-FGFR4 were selected with puromycin (2 µg/ml) for 2 days.

### Reverse transcriptase PCR (RT-PCR) and semiquantitative PCR

RNA was isolated using the RNeasy Mini Kit (Qiagen, Hilden, Germany) and reversely transcribed into cDNA using the AMV reverse transcriptase (Roche, Mannheim, Germany) and OligodT-Primer (Gibco, Germany). The RNA and cDNA concentration was measured using the NanoDrop ND1000 (PeqLab, Erlangen, Germany) system. For the semiquantitative PCR amplification the following primers (MWG, Ebersberg, Germany) have been used: 5'-ACCACAGTCCATGCCAT-CAC-3' (GAPDH forward)
and 5'-TCCACCACCCTG-TTGCTGTA-3' (GAPDH reverse),
5'-AGAATTCTGCCACCATGTCTCAGAGCAACC-3' (Bcl-xl forward) and 5'-GGG-TGATGTGGAGCTGGGATGTC-3' (Bcl-xl reverse). PCR products were subjected to electrophoresis on a 2% agarose gel and DNA was visualized by ethidium bromide staining.

### Cell lysis, immunoprecipitation and immunoblotting

Cell cultures were washed with PBS and incubated at 4°C with lysis buffer (50 mM HEPES/NaOH, pH 7.5, 150 mM NaCl, 1 mM EDTA, 10% glycerol and 1% Triton X-100) supplemented with phosphatase and protease inhibitors (10 mM sodium pyrophosphate, 1 mM PMSF, 2 mM sodium orthovanadate, 10 µg/ml aprotinin). Cellular debris was removed by centrifugation. Protein concentration measurements were performed using the Micro-BCA-Protein-Assay Kit from Pierce (Bonn, Germany). For immunoprecipitations, whole-cell lysates were combined with antibody and 30 µl of protein A- or G-sepharose slurry (GE Healthcare). The samples were incubated for 3 h on a rotation wheel at 4°C. The precipitates were washed three times with HNTG buffer (20 mM HEPES/NaOH, pH 7.5, 150 mM NaCl, 10% glycerol, 0.1% Triton X-100 and 10 mM sodium pyrophosphate) suspended in 3 x SDS sample buffer, boiled for 3 min and subjected to SDS-PAGE. For Western blot analysis, proteins were transferred to nitrocellulose membranes and incubated with the appropriate antibodies. Signals were developed via an enhanced chemiluminescence detection system (ECL, Perkin Elmer, Wellesley, MA). Before reprobing, membranes were stripped with 65 mM Tris/HCl pH 6.8 buffer containing 2% SDS at 50°C for 1 h.

### RNA interference

Transfection of 21 nucleotide siRNA duplexes (Ambion, Huntington, Cambridge, UK) for targeting endogenous genes was carried out using Lipofectamine 2000 (Invitrogen) and 0.84 µg of siRNA duplexes per 6-well plate as described by Elbashir et al. 2001.Transfected cells were assayed on indicated timepoints. Sequences of siRNA used were:
FGFR4 5'-GGCUCUUCCGGCAAGUCAAtt-3' and GL2-Luciferase 5'-CGUACGCGGAAUACUU-CGAtt-3'. Specific silencing of FGFR4 was confirmed by immunoblot analysis, as described before.

### Cell Death Assays

Cell death was measured by counting the percentage of hypodiploid cells using flow cytometry as described previously (Nicoletti, I., Migliorati, G., Pagliacci, M. C., Grignani, F., and Riccardi, C. (1991) J. Immunol. Methods 139, 271-279)). Propidium iodide fluorescence was measured in the FL2 channel on a FACSCalibur (BD Biosciences). Cell culture supernatant and trypsinized cells were pooled.

### cDNA Array Hybridization

Radioactive labeling of the cDNAs was achieved using the Megaprime-DNA labeling kit (Amersham Biosciences) and 50 µCi of [α-³³P]ATP per reaction. The labeled cDNA was purified via the Nucleotide Removal Kit from Qiagen and incubated with 0.5 mg/ml COT-DNA (Invitrogen) in hybridization buffer (5x SSC, 0.1% SDS) for 5 min at 95 °C and 30 min at 68 °C to block repetitive sequences in the cDNA. The cDNA was added to pre-warmed (68 °C) hybridization buffer containing 100 µg/ml tRNA (baker's yeast, Roche Applied Science). The cDNA arrays were incubated in pre-hybridization buffer (5x Denhardt's, 5x SSC, 100 mM NaPO₄, 2 mM Na₄P₂O₇, 100 µg/ml tRNA) for 4 h and subsequently with the labeled cDNA in hybridization buffer for 16 h. The cDNA was removed, and the cDNA arrays were washed with increasing stringency, dried, and exposed on phosphorimaging plates (Fuji). The plates were read on a FujiBas2500 phosphorimaging device, and the raw spot values (volume) were determined using ArrayVision (RayTest, Canada).

### Results

### Increased expression of FGFR4 in DXR-treated MDA-MB453 clones

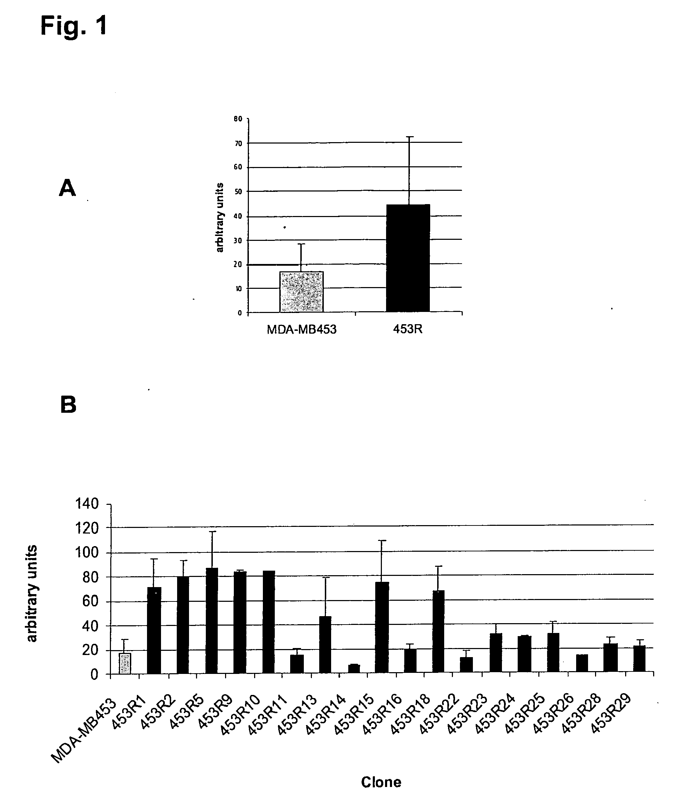

We performed an assay to identify antiapoptotic mechanisms in the breast cancer cell line MDA-MB 453. Therefore cells were treated with DXR and surviving clones were isolated. On average a 2-fold upregulation of FGFR4 mRNA was found in clones surviving the DXR treatment when the gene expression profiles generated by array analysis were compared (Fig. 1A). About half of the clones showed an up to 4-fold increase in FGFR4 mRNA-levels whereas others showed weak or no increase (Fig. 1 B). The FGFR4 expression levels were validated by semiquantitative PCR and quantified.

### Fgfr4 expression affects chemoresistance of cancer cells

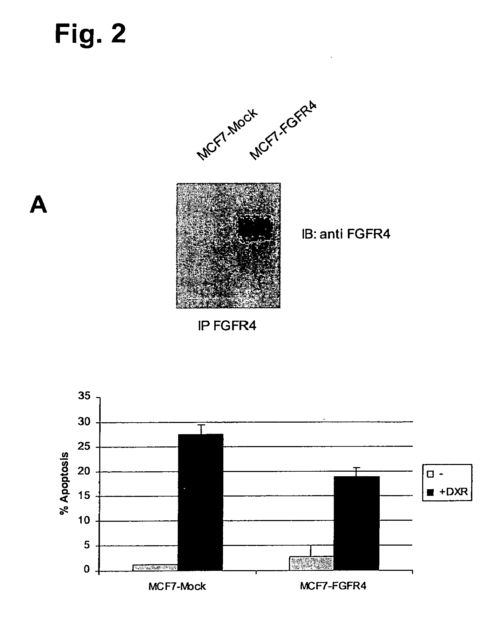

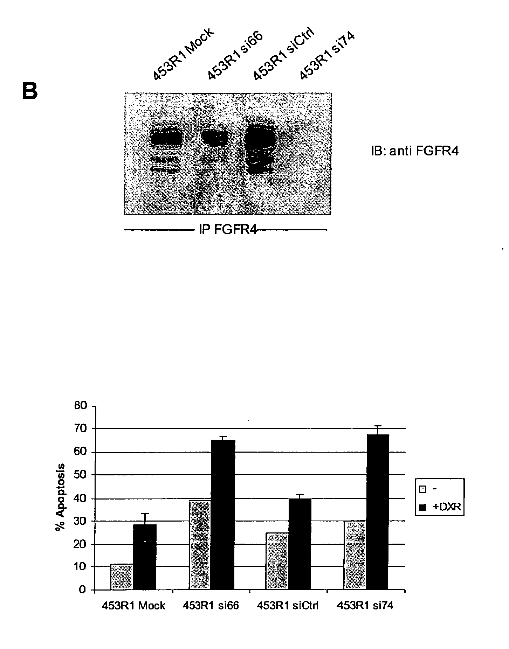

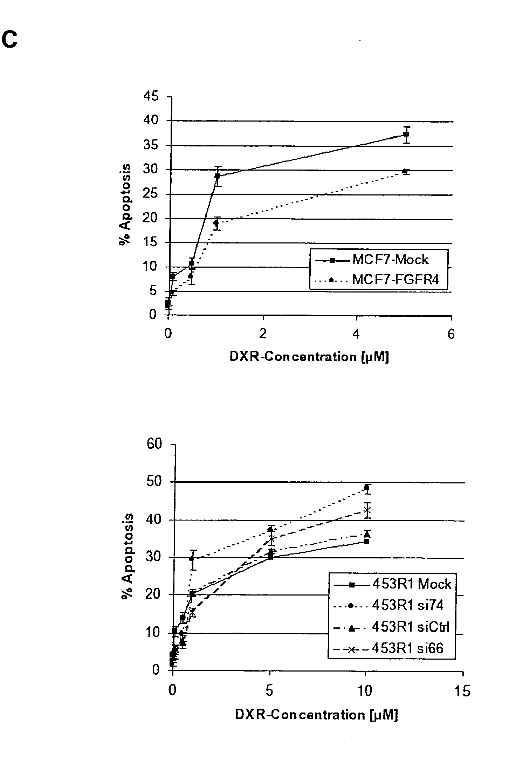

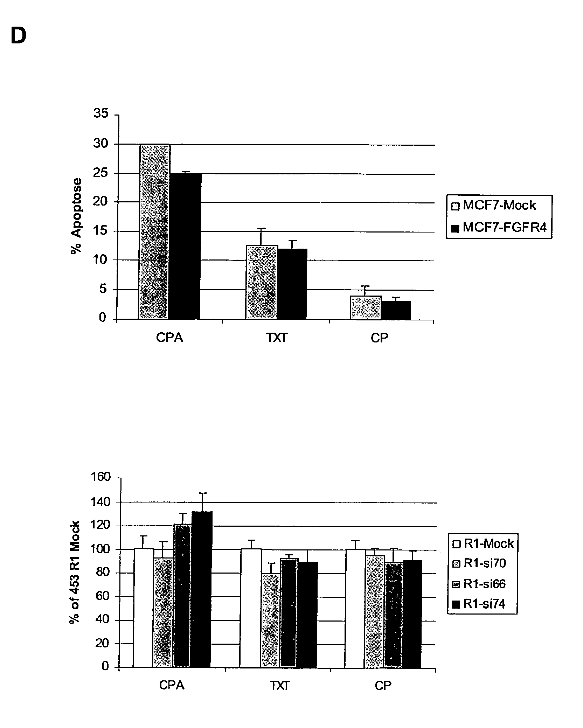

To further analyse the involvement of FGFR4 in antiapoptotic mechanisms we stably expressed the Fgfr4 gene in the breast cancer cell line MCF7 (Fig. 2A). Proliferation of these cells was not affected in normal culture conditions. However upon DXR treatment MCF7-Fgfr4 cells showed a faster proliferation rate than MCF7. Because of this decreased sensitivity to DXR in ectopic Fgfr4 expressing cells, we analysed the percentage of apoptotic cells by flow cytometry. Compared to parental MCF7 cells, MCF7-Fgfr4 expressing cells showed a reduced rate of apoptosis (Fig. 2A).

To further study the role of Fgfr4 in chemoresistance we utilised two of the resistant MDA-MB453 clones (453R1 and 453R9) and the kidney carcinoma cell line 769-P for Fgfr4 stable knockdown by two different FGFR4-siRNAs (si66 and si74). Here the siRNAs 66 and 74 could knockdown the receptor expression efficiently in 453R1, 769-P (Fig. 2B) and 453R9 cell lines. These cells exhibited an increase in apoptosis rate upon DXR treatment when compared to the Mock or ctrl-siRNA infected cells (Fig. 2B).

In order to show that the increased sensitivity against chemotherapeutic drugs is not only present at certain concentrations of DXR we analyzed the apoptosis rate of MCF7 cells ectopically expressing FGFR4 and 453R1 cells with FGFR4 knockdown at different DXR concentrations. In MCF7-Fgfr4 cells apoptosis was reduced at different DXR concentrations. Consequently the FGFR4 knockdown cells showed higher apoptosis rates compared to not infected cells (Fig 2C). Similar results were obtained with DXR treatment of the endogenously FGFR4 expressing kidney carcinoma cell line 769-P upon FGFR4 knockdown (Fig. 3).

Finally we analysed if the ectopic expression of Fgfr4 in MCF7 or a FGFR4 knockdown in 453R1 cells affect sensitivity against other chemotherapeutic drugs. Interestingly, only in the case of Cyclophosphamide (CPA) a reduced apoptosis sensitivity could be observed in FGFR4 overexpressing cells treated with different chemotherapeutic drugs. As expected 453R1 siRNA 66 and 74 cells exhibit a higher apoptosis rate than the control cells when treated with CPA (Fig 2D). No effect could be observed when cells were treated with Taxotere (TXT) and Cisplatin (CP). Altogether these experiments show that overexpression of FGFR4 increases apoptosis resistance whereas a stable knockdown of FGFR4 sensitizes cancer cells towards chemotherapeutic drug treatment.

### Knockdown of FGFR4 affects MAPK signalling pathways

To study the signalling pathways governed by FGFR4 we utilised different breast cancer cell lines ZR75-1 and BT474, which are endogenously expressing high amounts of FGFR4. The transient knockdown with synthetic siRNA directed against Fgfr4 (siFGFR4) showed reduction in phospho Erk levels at 96h hours (Fig. 4). The Akt phosphorylation was not affected by the knockdown and stayed activated at all time points (data not shown). Therefore we conclude that FGFR4 is regulating MAPK activation rather than Akt. Apoptosis rate was also affected by transient knockdown of the FGFR4 on different timepoints (Fig. 4B).

### Gene expression analysis of ectopic FGFR4 expressing cells reveals increased Bcl-xl levels

To gain further insight into the molecular mechanism mediating the chemoresistance of FGFR4 overexpressing cell lines we performed a gene expression array analysis of MCF7 cells ectopically expressing FGFR4. Here we could detect the Bcl-xl expression to be 2-fold upregulated due to the FGFR4 overexpression (Fig 5A). We validated the differences in expression levels of Bcl-xl by semiquantitative PCR. Bcl-xl protein was only found in the FGFR4 expressing MCF7 cell lines (Fig. 5B). To confirm that Bcl-xl expression is dependent on FGFR4 we transiently knocked down the Fgfr4 in different cell lines and analyzed the expression changes of both proteins. Here, we could show that Bcl-xl expression is suppressed with FGFR4 knockdown. These results showed that Bcl-xl expression is dependent on FGFR4 expression and could be suppressed by FGFR4 knockdown. As MAPK are very important regulators of Bcl-xl expression, MCF7-FGFR4 cells were treated with UO126, a specific MEK inhibitor. The semiquantitative PCR showed that UO126 treatment abolished Bcl-xl expression (Fig. 5D). Therefore we conclude that Bcl-xl expression is dependent on FGFR4 expression and MAPK activity.

### A Fgfr4 blocking antibody inhibits FGF19 induced MAPK activation

Next we wanted to address the question if the enhanced chemoresistance is linked with the Fgfr4 activity in the different cancer cell lines. For this purpose a Fgfr4 blocking antibody (10F10) was used. We stimulated various breast cancer cell lines, which endogenously express Fgfr4, with its specific ligand FGF19. When FGF19 treated, p-Erk-levels were increased compared to not stimulated control cells. After preincubation of the cell lines with the Fgfr4 blocking antibody and subsequent FGF19 stimulation reduced p-Erk levels compared to sole FGF19 treated cancer cell lines could be detected (Fig. 6 A). In these experiments we clearly could show that the Fgfr4 blocking antibody (10F10) is inhibiting the Fgfr4 mediated downstream signalling.

To verify if the blocking of the Fgfr4 receptor activity results in increased sensitivity to chemotherapeutic drugs, we incubated the breast cancer cell lines MDA-MB-361 and BT474 with DXR, VSV/10F10 antibody or in different combinations and measured the apoptosis rate after 48h of DXR-treatment. Here we could demonstrate that the blocking of FGFR4 by 10F10-antibody resulted in increased apoptosis rate due to DXR-induction whereas the VSV-antibody incubation had no effect on apoptosis (Fig. 6 B).

### The FGFR4 blocking antibody (10F10) reduces the chemoresistance of DXR-treated cancer cells

In cDNA-microarray experiments we could observe that Fgfr4 is highly expressed in about 30 percent of the available breast cancer cell lines (Fig. 7A). High expression of the receptor was observed in the cell lines BT-474, ZR75-1, MDA-MB-361 and MDA-MB-453. Therefore these cell lines were used to inhibit FGFR4 signaling and to sensitize the cancer cells for chemotherapy. We compared the apoptosis rates of FGF19/DXR treated cancer cell lines with cell lines additionally treated with FGFR4 blocking antibody. DXR/FGF19 treatment of the different cell lines induced apoptosis in a low to medium extent whereas the additional treatment with FGFR4 blocking antibody significantly further increased the apoptosis rates (Fig. 7 B): in MDA-MB-361 about 40%, in ZR75-1 about 29% and BT-474 about 10%. In MDA-MB-453 cells we could not detect increased apoptosis with 10F10-AB treatment. This might be due to the constitutive activation of the FGFR4 in this cell line which could not be blocked by the 10F10 antibody (data not shown). However in these experiments we could demonstrate that FGFR4 activity supports resistance of cancer cells to DXR whereas inversely the inhibition of FGFR4 activation by a FGFR4 blocking antibody (10F10) leads to increased chemosensitivity of cancer cells.

We finally analyzed the efficacy of the blocking Ab 10F10 in cancer cell lines of other origin than breast tissue. As an example we used the 769-P kidney carcinoma and the TE671 glioblastoma cell lines. Treatment with the blocking antibody increased apoptosis rate in these cells.

## Claims

1. Use of an FGFR4 inhibitor for the manufacture of a medicament for the prevention, alleviation or/and treatment of a hyperproliferative disorder in combination with a further therapeutic procedure or/and a further therapeutic agent.

2. The use of claim 1, wherein the further therapeutic procedure or/and agent is an apoptosis-inducing therapeutic procedure or/and agent.

3. The use of claim 1 or 2 wherein the hyperproliferative disorder is associated with FGFR4 overexpression.

4. The use of any of the claims 1 to 3, wherein the hyperproliferative disorder is selected from cancers, such as kidney, bladder, brain, esophagus, gastric, pancreas, small intestine, colon, breast, lung, liver, spleen, thyroid, pituitary, adrenal, ovarian, cervix, testis, prostate cancer, glioma, melanoma, or/and leukemia.

5. The use of any of the claims 1 to 4 wherein the hyperproliferative disorder is at least partially therapy-resistant.

6. The use of any of the claims 1 to 5 wherein the hyperproliferative disorder is at least partially resistant against an apoptosis-inducing therapeutic procedure or/and agent.

7. The use of any of the claims 1 to 6 wherein the further therapeutic procedure is radiation therapy.

8. The use of any of the claims 1 to 7 wherein the further therapeutic agent is selected from cytostatic, cytotoxic or/and chemotherapeutic agents.

9. The use of claim 8 wherein the cytostatic, cytotoxic or/and chemotherapeutic agent is selected from doxorubicin, taxanes, platinum compounds, nucleoside analogues, mitomycin D, etoposide, cyclophosphamide, topoisomerase inhibitors and proteins such as anti-tumor antibodies.

10. The use of any of the claims 1 to 9, wherein the hyperproliferative disorder is associated with overexpression or/and hyperactivity of BclX_{L} or/and MAP kinases.

11. Use of an FGFR4 inhibitor for the manufacture of a medicament for increasing the efficacy of a therapy or/and agent against a hyperproliferative disorder.

12. Use of an FGFR4 inhibitor for the manufacture of a medicament for increasing the sensitivity of a hyperproliferative disorder against radiation and/or medicament treatment.

13. The use of claim 11 or 12, wherein the hyperproliferative disorder is cancer.

14. The use of any one of the claims 1 to 13 wherein the FGFR4 inhibitor is a direct FGFR4 inhibitor.

15. The use of any one of the claims 1 to 13 wherein the FGFR4 inhibitor is an indirect FGFR4 inhibitor.

16. The use of any one of the claims 1 to 15 wherein the FGFR4 inhibitor is selected from anti-sense molecules, ribozymes, siRNA molecules, antibodies and antibody fragments, soluble FGFR4 molecules, antagonistic FGFR4 ligand analogues, peptides and low-molecular weight compounds.

17. The use of any of the claims 1 to 16, wherein the FGFR4 inhibitor is an FGFR4 antibody or a siRNA molecule directed against FGFR4 mRNA.

18. A pharmaceutical composition or kit comprising as active ingredients
(a) an FGFR4 inhibitor, and
(b) an agent for the prevention, alleviation or/and treatment of a hyperproliferative disorder, which is different from (a),
optionally together with a pharmaceutically acceptable carrier, diluent or/and adjuvant.

19. The pharmaceutical composition or kit of claim 18, wherein the agent (b) is an apoptosis-inducing agent.

20. The composition or kit of claim 18 or 19, wherein the inhibitor of (a) and the agent of (b) are provided in in the form of a single composition or in the form of at least two distinct compositions.

21. A method for preventing, alleviating or/and treating of a hyperproliferative disorder comprising administrating an effective amount of an FGFR4 inhibitor in co-administration with a further therapeutic procedure or/and a further therapeutic agent for a subject in need thereof.

22. The method of claim 21, wherein the further therapeutic procedure or/and agent is an apoptosis-inducing therapeutic procedure or/and agent.

23. The method of claim 21 or 22, wherein the hyperproliferative disorder is at least partially therapy resistant.

24. A method for the diagnosis of a hyperproliferative disorder, wherein a sample from a subject which may suffer from a hyperproliferative disorder is analysed for the expression of the FGFR4 gene.

25. A method for the diagnosis of a hyperproliferative disorder, wherein a sample from a subject which may suffer from a hyperproliferative disorder is analysed for the presence of polymorphisms in the FGFR4 gene.

26. A method for the diagnosis of a hyperproliferative disorder, wherein a sample from a subject which may suffer from a hyperproliferative disorder is analysed for the expression of the FGFR4 gene and for the presence of polymorphisms in the FGFR4 gene.

27. The method of claim 25 or 26, wherein the polymorphism is at a position encoding amino acid 388 of human FGFR4.

28. The method of any one of claims 24-27, wherein the hyperproliferative disorder is a cancer.

29. The method of claim 28, wherein the cancer is a non-metastatic cancer.

30. The method of claim 28, wherein the cancer is a metastatic cancer.

31. The method of any one of claims 24-30 further comprising determining the apoptosis resistance status of the hyperproliferative disorder.

32. The method of any one of claims 24-31 further comprising designing a therapeutic regimen for the treatment of the hyperproliferative disorder based on the results of the diagnosis.

33. The method of claim 32, wherein the hyperproliferative disorder is a non-metastatic cancer associated with FGFR4 overexpression and wherein the therapeutic regimen comprises the administration of an FGFR4 inhibitor in combination with a further therapeutic procedure or/and a further therapeutic agent.

34. The method of claim 33, wherein the non-metastatic cancer is associated with the FGFR4-388 Gly or/and the FGFR4-388 Arg allele.

35. The method of claim 32, wherein the hyperproliferative disorder is a metastatic cancer associated with the FGFR4-388 Arg allele and optionally with FGFR4 overexpression and wherein the therapeutic regimen comprises the administration of an FGFR4 inhibitor in combination with a further therapeutic procedure or/and a further therapeutic agent.

36. The method of claim 32, wherein the hyperproliferative disorder is a metastatic cancer associated with the FGFR4-388 Gly allele and wherein the therapeutic regimen comprises administration of an FGFR4 agonist optionally in combination with a further therapeutic procedure or/and a further therapeutic agent.

37. A screening method for identifying or/and characterizing a compound suitable for reducing the apoptosis resistance of a hyperproliferative disorder comprising determining, if the compound is an FGFR4 inhibitor.

38. The screening method of claim 37, wherein the hyperproliferative disorder is an at least partially therapy resistant hyperproliferative disorder.

39. The screening method of claim 37 or 38, comprising a molecular or/and cellular assay.

40. The screening method of any one of claims 37 to 39 wherein identifying the FGFR4 inhibitor comprises the steps
(a) contacting at least one compound with FGFR4, and
(b) determining FGRF4 activity in the presence of the compound.
